Europäisches Patentamt

European Patent Office

Office européen des brevets

(19)

(11) Veröffentlichungsnummer: **0 011 244**
**A1**

# EUROPÄISCHE PATENTANMELDUNG

(12)

(21) Anmeldenummer: **79104392.0**

(22) Anmeldetag: **08.11.79**

(51) Int. Cl.³: **C 07 D 307/08**

(30) Priorität: **11.11.78 DE 2848979**

(43) Veröffentlichungstag der Anmeldung:
**28.05.80 Patentblatt 80/11**

(84) Benannte Vertragsstaaten:
**AT BE CH DE FR GB IT LU NL SE**

(71) Anmelder: **HOECHST AKTIENGESELLSCHAFT**
**Zentrale Patentabteilung Postfach 80 03 20**
**D-6230 Frankfurt/Main 80(DE)**

(72) Erfinder: **Kümmerle, Kurt, Dr.**
**Unter den Eichen 1**
**D-6233 Kelkheim (Taunus)(DE)**

(72) Erfinder: **Heise, Hartmut, Dr.**
**Am Rehsteig 8**
**D-6232 Bad Soden am Taunus(DE)**

(54) **Verfahren zur Gewinnung von im wesentlichen wasserfreiem Tetrahydrofuran.**

(57) Roh-Tetrahydrofuran, das neben Tetrahydrofuran und Wasser in untergeordneten Mengen im wesentlichen primäre aromatische Amine als Verunreinigungen enthält, läßt sich durch eine Extraktiv-Destillation aufarbeiten. Als Zusatzstoff wird Äthylenglykol oder 2-Aminoäthanol eingesetzt. Tetrahydrofuran läßt sich am Kopf der benutzten Kolonne abziehen. Es ist frei von Wasser und Amin. Der verwendete Zusatzstoff läßt sich aus dem Sumpf isolieren und erneut einsetzen.

EP 0 011 244 A1

- 1 -

Verfahren zur Gewinnung von im wesentlichen wasserfreien
Tetrahydrofuran

Die vorliegende Erfindung betrifft ein Verfahren zur Aufarbeitung von Tetrahydrofuran/Wasser-Gemischen, die bei der katalytischen Reduktion aromatischer Nitroverbindungen anfallen.

Für die Abtrennung von Tetrahydrofuran aus Tetrahydrofuran/Wasser-Mischungen sind verschiedene Verfahren vorgeschlagen worden. Gemäß DT-PS 713 565 wird wasserfreies Tetrahydrofuran erhalten, indem man das Ausgangsgemisch zunächst so weit rektifiziert bis das Azeotrop vorliegt (das Azeotrop enthält bei 1 bar ca. 19 Mol-% Tetrahydrofuran). Das Azeotrop wird dann mit Natriumchlorid und anschließend mit Kaliumhydroxid behandelt. Die letzten Reste Wasser lassen sich durch Abdestillation eines Vorlaufes entfernen. Tetrahydrofuran bildet die Hauptfraktion.

Gemäß DT-PS 740 187 kann man Tetrahydrofuran/Wasser-Gemische auch mit höher siedenden Kohlenwasserstoffen, Halogenkohlenwasserstoffen oder Nitrokohlenwasserstoffen vermischen, anschließend die entstandenen beiden flüssigen Phasen voneinander trennen und schließlich die nicht-wässrige Phase fraktioniert destillieren, wobei als Vorlauf noch wasserhaltiges Tetrahydrofuran anfällt, während der Hauptlauf wasserfreies Tetrahydrofuran darstellt.

Ein ähnliches Verfahren ist in der GB-PS 630 149 angegeben. Die erste Verfahrensstufe ist hier ebenfalls eine Flüssig-Flüssig-Extraktion, jedoch mit dem Unterschied, daß anstelle eines hydrophoben nun ein hydrophiles Solvens, nämlich Glycerin verwendet wird.

Die Entwässerung des binären Azeotrops Tetrahydrofuran/Wasser kann ferner durch Addukt-Bildung mittels Calciumchlorid erfolgen, wobei eine Additionsverbindung aus Tetrahydrofuran/Calciumchlorid/Wasser entsteht (US-PS 2 795 591).

Die Kombination einer Flüssig-Flüssig-Extraktion mit einer Destillation ist jedoch apparativ aufwendig, die Verwendung von reagierenden Hilfsstoffen (Calciumchlorid) unwirtschaftlich.

Es bestand daher die Aufgabe ein Verfahren zur Gewinnung von im wesentlichen wasserfreiem Tetrahydrofuran aus einem Roh-Tetrahydrofuran, das neben Tetrahydrofuran und Wasser in untergeordneten Mengen im wesentlichen primäre aromatische Amine als Verunreinigungen enthält, destillativ aufzutrennen.

Die vorliegende Erfindung löst diese Aufgabe. Das erfindungsgemäße Verfahren ist dadurch gekennzeichnet, daß man das Roh-Tetrahydrofuran einer Extraktivdestillation mit Äthylenglycol oder Aminoäthanol als Zusatzstoff unterwirft.

Es ist zwar aus Chemical Abstracts Band 69 (1968) 61931a bekannt, daß sich Tetrahydrofuran/Wasser-Gemische durch extraktive Destillation auftrennen lassen, wenn Äthylenglykol als Zusatzstoff verwendet wird. Es konnte jedoch nicht vorhergesehen werden, daß dieses Trennverfahren auch bei Anwesenheit unterschiedlicher aromatischer Amine zufriedenstellend verlaufen würde.

Die Menge an einem der genannten Zusatzstoffe beträgt bei Atmosphärendruck mindestens 75 Mol-% (Rest: Tetrahydrofuran und Wasser).

Der Gehalt des Ausgangsgemisches an primärem aromatischem Amin soll bei 0 bis 6, vorzugsweise 0,5 bis 5, insbesondere 2 bis 4 Gew.-% liegen.

Das bei der Extraktivdestillation anfallende Sumpfprodukt (Amin + Hilfsstoff + Wasser) läßt sich durch normale Rektifikation in Wasser als Kopfprodukt und in Amin/Zusatzstoffhaltiges Sumpfprodukt trennen und letzteres, gegebenenfalls nach Abtrennung des aromatischen Amins, wieder in die Extraktivdestillations-kolonne zurückführen.

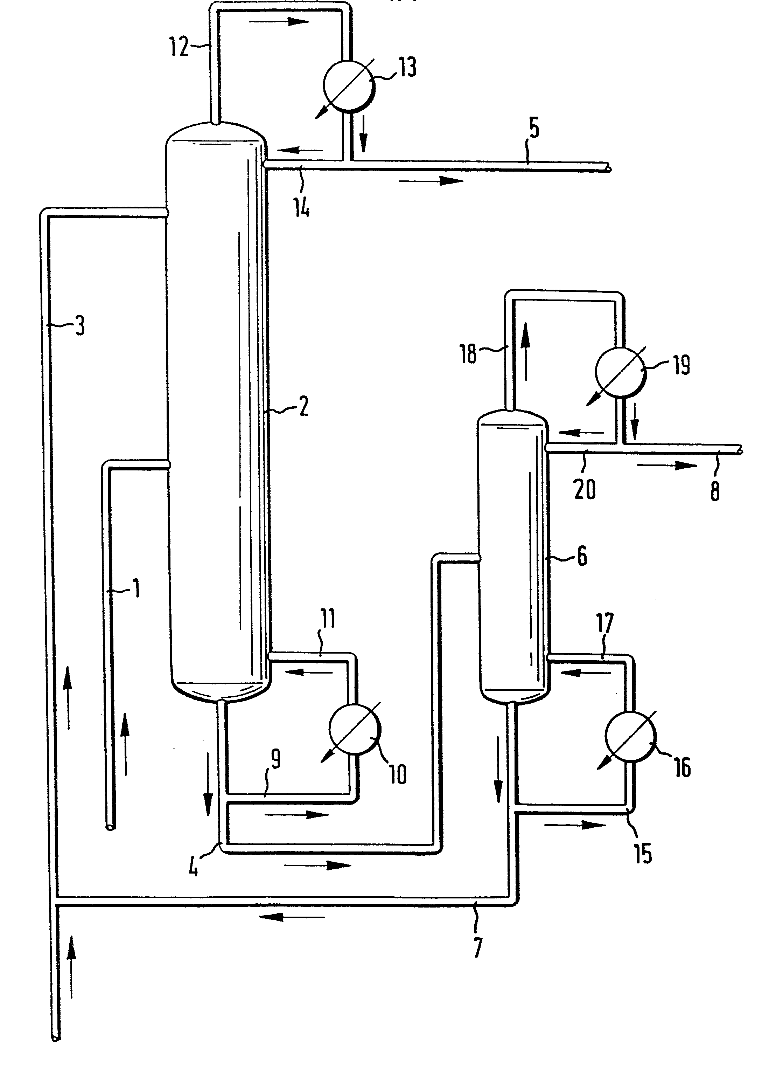

Das Trennverfahren kann mit der in der Figur dargestellten Anlage durchgeführt werden.

Das zu trennende Rohgemisch wird dampfförmig oder flüssig durch Leitung (1) dem Mittelteil einer Extraktivdestillationskolonne (2) zugeführt, die in der Nähe ihres oberen Endes mit dem zu verwendenden Hilfsstoff durch Leitung (3) beschickt wird. Am unteren Ende der Kolonne wird der Sumpf durch Leitung (4) abgezogen. Er besteht aus dem Zusatzstoff, beladen mit Wasser und Amin, ist aber nahezu frei von Tetrahydrofuran. Ein Teil dieses Sumpfes wird der Leitung (4) über Leitung (9) entnommen, in den Erhitzer (10) eingespeist, dort aufgeheizt und über Leitung (11) erneut in die Kolonne (2) zurückgeführt.

Am Kopf der Kolonne (2) wird über die Leitung (12) das nahezu wasserfreie, aminfreie und vom Zusatzstoff befreite Tetrahydrofuran abgezogen und im Kühler (13) kondensiert. Ein Teil des Kondensats läuft - in Abhängigkeit vom gewünschten Rücklaufverhältnis - über Leitung (14) in die Kolonne (2) zurück. Das restliche Tetrahydrofuran wird über Leitung (5) abgezogen. Durch entsprechende Einstellung des Rücklaufs und der aufgegebenen Menge an Zusatzstoff läßt sich eine optimale Trennung erreichen.

Das Gemisch aus Zusatzstoff, Amin und Wasser von Leitung (4) wird in eine einfache Destillationskolonne (6) eingespeist und dort vom Wasser befreit. Das Wasser geht dampfförmig am Kopf der Kolonne (6) über Leitung (18) ab und wird im Kühler (19) kondensiert. Ein geringer Teil Wasser kann, zur Einstellung des Rücklaufverhältnisses, über Leitung (20) in die Kolonne (6) zurückgeführt werden, der Rest wird über Leitung (8) ausgekreist. Am unteren Ende der Kolonne (6) wird über Leitung (7) der Zusatzstoff abgezogen. Ein Teil wird über Leitung (15) ausgeschleust, im Erhitzer (16) aufgeheizt und über Leitung (17) in die Kolonne zurückgeführt. Der Zusatzstoff von Leitung (7) wird in Leitung (3) eingespeist. Nach wiederholter Rückführung muß der durch Amin verunreinigte Zusatzstoff vor der Einschleusung in Kolonne (2) durch eine einfache Destillation (nicht gezeichnet) vom aromatischen Amin befreit werden.

Die folgenden Beispiele dienen der weiteren Erläuterung des erfindungsgemäßen Verfahrens.

Beispiel 1

Ein Gemisch aus gleichen Massenteilen Tetrahydrofuran und Wasser, enthaltend 3 % 4-Amino-2',4'-dichlordiphenyläther wurde einer selektiven Rektifikation mit Ethylenglykol als Zusatzstoff bei 760 Torr unterworfen. Die kontinuierlich arbeitende Rektifikationskolonne bestand aus einer Anreicherungszone mit ca. 15 Böden, einer selektiven Anreicherungszone mit ca. 45 Böden und einer normalen Anreicherungszone mit ca. 10 Böden. Bei einem stündlichen Zulauf von 180 g des Ausgangsgemisches, einer Konzentration des Zusatzstoffes von 85 Mol-% - auf das Gesamtgemisch bezogen - und einem Rücklaufverhältnis von ca. 1 fiel Tetrahydrofuran mit einem Reinheitsgrad größer 99,5 mass.-% als Kopfprodukt an. Das Sumpfprodukt, das frei von Tetrahydrofuran war, wurde in einer nachgeschalteten normalen Rektifikationskolonne praktisch wasserfrei erhalten.

Beispiel 2

"Mutterlauge", die aus der katalytischen Hydrierung aromatischer Nitroverbindungen stammt (Gehalt an primärem aromatischen Amin ca. 3 %) und deren Schlüsselkomponenten Tetrahydrofuran und Wasser - mit einem Wasseranteil von ca. 85 mass.-% - sind, wurde bei Normaldruck in Gegenwart von 2-Aminoethanol selektiv rektifiziert. Die selektive Rektifikationskolonne bestand aus einer Abreicherungszone mit

0011244

ca. 25 Böden, einer selektiven Anreicherungszone mit ca. 60 Böden und einer normalen Anreicherungszone mit ca. 15 Böden. Bei einem stündlichen Zulauf von 210 g des Ausgangsgemisches, einer Konzentration des Zusatzstoffes von 85 Mol-% auf das Gesamtgemisch bezogen - und einem Rücklaufverhältnis von ca. 1 konnte ein Kopfprodukt erhalten werden, dessen Gehalt an Tetrahydrofuran größer als 99,5 mass-% war. Im Sumpfprodukt ließ sich kein Tetrahydrofuran mehr nachweisen.

0011244

Patentansprüche:

1. Verfahren zur Gewinnung von im wesentlichen wasserfreiem Tetrahydrofuran aus einem Roh-Tetrahydrofuran, das neben Tetrahydrofuran und Wasser in untergeordneten Mengen im wesentlichen primäre aromatische Amine als Verunreinigungen enthält, dadurch gekennzeichnet, daß man das Roh-Tetrahydrofuran einer Extraktivdestillation mit Äthylenglycol oder 2-Aminoäthanol als Zusatzstoff unterwirft.

2. Verfahren nach Anspruch 1, dadurch gekennzeichnet, daß man das bei der Extraktivdestillation anfallende Sumpfprodukt durch normale Rektifikation in Wasser als Kopfprodukt und in Amin und Zusatzstoff enthaltendes Sumpfprodukt trennt und letzteres, gegebenenfalls nach Abtrennung des aromatischen Amins, wieder in die Extraktivdestillation zurückführt.

**Europäisches Patentamt**

# EUROPÄISCHER RECHERCHENBERICHT

| EINSCHLÄGIGE DOKUMENTE | | | KLASSIFIKATION DER ANMELDUNG (Int.Cl. ³) |
|---|---|---|---|
| Kategorie | Kennzeichnung des Dokuments mit Angabe, soweit erforderlich, der maßgeblichen Teile | betrifft Anspruch | |
| X | CHEMICAL ABSTRACTS,vol.64,nr.2,17-01 1966, Spalte 2056d Columbus, Ohio, US<br><br>& SU - A - 174 636 (V.A. SHNITKO) (07-09-1965)<br><br>  * Zusammenfassung * <br><br>-- | 1,2 | C 07 D 307/08 |
| X | CHEMICAL ABSTRACTS, vol. 77, Nr. 11 11-09-1972,Seite 470, ref. 75115q Columbus, Ohio, US<br><br>& JP - A - 72 20 009 (NISSAN CHE-MICAL INDUSTRIES)(07-06-1972)<br><br>  * Zusammenfassung * <br><br>-- | 1,2 | |
| DX | CHEMICAL ABSTRACTS, vol. 69, Nr. 16, 14-10-1968; Seite 5801, ref. 61931a Columbus, Ohio, US V.A. SHNITKO et al.: "Liquid-vapor equilibrium in tetrahydrofuran-water and tetrahydrofuran-ethylene glycol systems and a method for dehydrating tetrahydrofuran"<br><br>& Zh. Prikl.Khim. (LENINGRAD) 1968, 41 (6), 1305-13.<br><br>  * Zusammenfassung * <br><br>---- | 1,2 | |

**RECHERCHIERTE SACHGEBIETE (Int. Cl. ³)**

C 07 D 307/08

**KATEGORIE DER GENANNTEN DOKUMENTE**

X: von besonderer Bedeutung
A: technologischer Hintergrund
O: nicntschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patent-familie, übereinstimmendes Dokument

Der vorliegende Recherchenbericht wurde für alle Patentanspruche erstellt.

| Recherchenort | Abschlußdatum der Recherche | Prüfer |
|---|---|---|
| Den Haag | 08-02-1980 | ALLARD |

EPA form 1503.1  06.78